Europäisches Patentamt

**European Patent Office**

Office européen des brevets

(11) Publication number: **0 036 915**
A1

## (12) EUROPEAN PATENT APPLICATION

(21) Application number: 80300972.9

(22) Date of filing: 28.03.80

(51) Int. Cl.³: **C 02 F 3/28**
**B 65 D 88/76, B 65 D 88/34**
**A 01 C 3/02**

(43) Date of publication of application:
07.10.81 Bulletin 81/40

(84) Designated Contracting States:
AT BE CH DE FR IT LU NL SE

(71) Applicant: **COMMONWORK ENTERPRISES LIMITED**
**Bore Place**
**Chiddingstone Edenbridge, Kent TN8 7AR(GB)**

(72) Inventor: **Dodson, Christopher**
**c/o Helix Multiprofessional Serv.**
**Mortimer Hill Mortimer, Reading(GB)**

(74) Representative: **Silverman, Warren et al,**
**HASELTINE LAKE & CO. 28 Southampton Buildings**
**Chancery Lane**
**London WC2A 1AT(GB)**

(54) Capping device and slurry digester comprising the same.

(57) A modular capping arrangement which enables capping of an anaerobic slurry digester of rectangular form to be effected readily irrespective of the width of the digester comprises a plurality of elongate capping members (108) of like width placed in side-by-side arrangement. The capping members define therebetween channels which are upwardly open. The capping members (108) are formed with gas outlets in upper regions thereof for removal of gas produced during slurry digestion and are weighed down on the slurry by ballast means (118) positioned in the upwardly open channels. The ballast means act to keep the capping members in their side-by-side arrangement when positioned on the digester and floating on a body of slurry and when being lifted therefrom by gas under pressure.

EP 0 036 915 A1

FIG.4.

-1-

# CAPPING DEVICE AND SLURRY DIGESTER COMPRISING THE SAME

This invention relates to a capping arrangement for an anaerobic slurry digester of substantially rectangular horizontal cross-section, the capping arrangement being intended to float on slurry in the digester and be lifted from the slurry by gas under pressure produced in the digester and comprising a plurality of ballasted elongate capping members arranged side-by-side.

Slurry digesters for use in the purification of sewage slurries generally take the form of massive concrete containers located above ground. Whilst the slurry digesters of such size may be required for handling urban sewage, they do not require such size when employed in rural locations, particularly on farms where they may be required to handle slurry resulting, for example, from the hosing out of animal sheds. Moreover, in such rural environments, massive concrete settling tanks are visually unattractive.

Insofar as slurry digesters intended for rural use in particular have hitherto been devised, including the so-called Ghoba digesters, German domestic sewage digesters and Chinese mass applied underground systems which are allied to small scale industrial plants, the most striking fact which has arisen is that almost every system is in practice a "one off". None of the systems described for small scale use is in any way adaptable for really large scale energy farms or industrial effluent treatment without considerable problems in structural engineering and mechanical handling.

United States Patent No. 3,933,628 discloses an anaerobic slurry digester for use in rural locations having a digester basin covered and sealed by a liquid-filled pond heated with solar energy. The pond is covered with a translucent roof capable of transmitting solar energy, this roof co-operating with the liquid-filled pond therebeneath to produce an artificial environment which is operative to help maintain a condition of stable equilibrium within the digester. With such an arrangement, it is necessary for the cover which supports the pond to be made to size and, if servicing of the tank, for example for removal of scum is required, it is necessary for supply of slurry to the tank to be stopped and the entire cover removed before the necessary work on the interior of the tank can be carried out. Because of the nature of the material being handled, the removal of the entire cover can be a singularly unpleasant task.

United States Patent No. 2,986,302 discloses a self-adjusting floating roof for storage reservoirs. This roof comprises a plurality of elongated sections in side-by-side arrangement across the width of the reservoir and which co-act together. The sections comprise a main section intended to lie above the flat base of the reservoir which extend over a major part of the width and, flanking it on either side, a small number of sections of trapezoidal form having sloping base surfaces corresponding to the slope of side walls of the reservoir and which are intended to lie parallel and adjacent to the sloping side walls of the base of the reservoir when the reservoir contents are at a low level. As the level of the contents rises, the side sections will gradually be floated off until eventually the upper surfaces of all the sections are level. At all times, the contents of the reservoir remain covered owing to the manner of co-acting together of the roof section. The roof sections adjacent the periphery of the reservoir alone are ballasted, the ballasting being provided within the cover sections themselves and serving to prevent the side roof sections from being floated off too rapidly as liquid level within the reservoir increases. In fact, the reservoir according to United States Patent No. 2,986,302 is not to be compared with an anaerobic slurry digester. The cover is presumably to prevent vapours from rising from the reservoir and conversely to prevent foreign bodies entering thereinto. Such vapour would not normally include gases in a quantity as will be produced continuously in a slurry digester constantly being fed with slurry and having treated slurry removed therefrom. It is suggested that in fact what is contemplated here is a cover for oil storage tanks. Hence, there is no provision made for the removal of gases from above the liquid undergoing storage. In fact, there is insufficient space between the liquid and the roof for gas in any substantial volume to accumulate. Moreover, the arrangement according to United States Patent No. 2,986,302 is of limited applicability in that the main roof section has to be constructed in accordance with predetermined dimensions of the base of the reservoir.

It is an object of this invention to provide an anaerobic slurry digester system which can be constructed in a wide variety of sizes utilizing the same constructional principle in each case.

It is a further object of the invention to provide a modular capping arrangement for a slurry tank, which capping arrangement can be constructed from a plurality of standard modules in accordance with the size of the tank.

The present invention provides a capping arrangement of the type first mentioned herein which is characterised in that it comprises an array of capping members of like width in a modular arrangement, which capping members are of inverted channel form defining therebetween channels which are upwardly open, the capping members being formed with gas outlets in upper regions thereof and ballast means being positioned in the upwardly open channels and acting to keep the capping members in their side-by-side arrangement when positioned on the digester and floating on a body of slurry and when being lifted therefrom by gas under pressure.

Such a capping arrangement is intended for use in an anaerobic slurry digester which comprises a basin of substantially rectangular horizontal cross-section and having a substantially horizontal upper periphery, inlet means for supply of slurry to the basin below the periphery and means for removal of sludge from the basin, the capping arrangement employed being sized to lie within the basin with its outer margin adjacent the periphery thereof and disposed in an upper region of the basin.

A slurry digester incorporating such a capping arrangement is especially useful where relatively small scale slurry digestion is required as on farm sites. In preferred practice, the basin is an excavated trough of substantially rectangular horizontal cross-section in the earth which is lined to prevent liquid seepage. The lining then employed so as to prevent water seepage therefrom may be provided by a rubber or plastics (e.g. butyl rubber or polyvinylchloride) sealing sheet laid on a sand lining. Such a trough when provided on a farm site may be of a size such as can be readily excavated by conventional excavating equipment. Alternatively, the basin may be an above-ground construction. In general, however, reference will be made hereinafter to troughs as aforesaid, although it is not intended that this invention should be limited thereto.

The capping arrangement is essentially of modular form and can be readily adapted in accordance with the size of basin to be capped by provision of a suitable number and/or length of capping members. One form of capping arrangement which may be employed comprises a plurality of elongate members of substantially hemi-cylindrical cross-section and having at their lateral margins flange sections at which the capping members adjoin when juxtaposed in parallel, the members being formed with gas relase outlets in upper regions thereof. Ballasting of the capping members may be provided by means of elongate tanks positioned between adjacent capping members and cummunicable with the outlets, which elongate tanks are adapted for supply of ballasting water thereto and removal of such water therefrom.

In an alternative form of capping arrangement, the capping members have upstanding lateral walls formed with shoulders on which rest ballast members which are substantially of letter T-shape in transverse cross-section. The shoulders are preferably profiled and the ballast members correspondingly shaped so that the ballast members coact with the shoulders to interconnect and hold together adjacent capping members.

With the first described preferred form of capping arrangement, ballasting is effected by supply of water to the elongate tanks in accordance with the amount of ballasting required. Whilst this allows for flexibility of operation, it makes for a more complicated system to operate. In contrast, with the second form of construction, solid ballasting may be employed in the upwardly open channels. By suitable shaping of the elongate capping members in their marginal regions, it is possible to employ preformed ballast members, formed particularly of concrete, to couple together adjacent capping members.

For a better understanding of the invention and to show how the same may be carried into effect, reference will now be made, by way of example only, to the accompanying drawings, wherein:

Figure 1 is a plan view of one form of slurry digester according to the invention:

Figure 2 is a cross-section through the slurry digester of Figure 1 at II-II showing the capping arrangement thereof;

Figure 3 is a plan view of an alternative form of slurry digester according to the invention;

Figure 4 is a cross-section through the slurry digester of Figure 3 at IV-IV showing the capping arrangement thereon; and

Figure 5 is a cross-section through the slurry digester of Figure 3 at V-V.

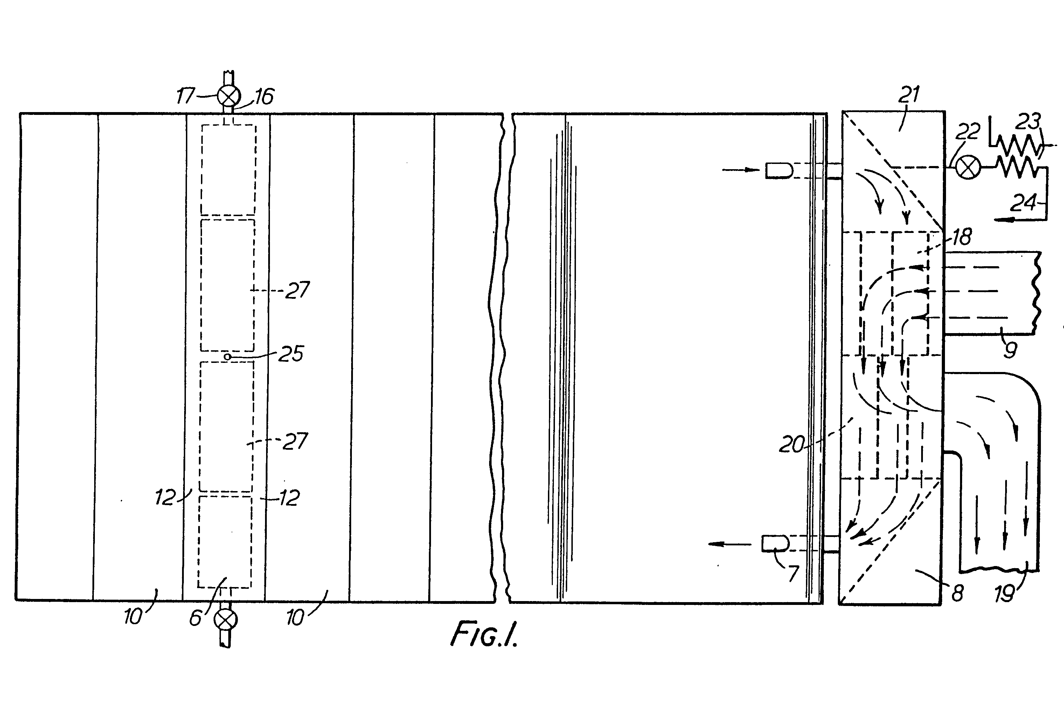

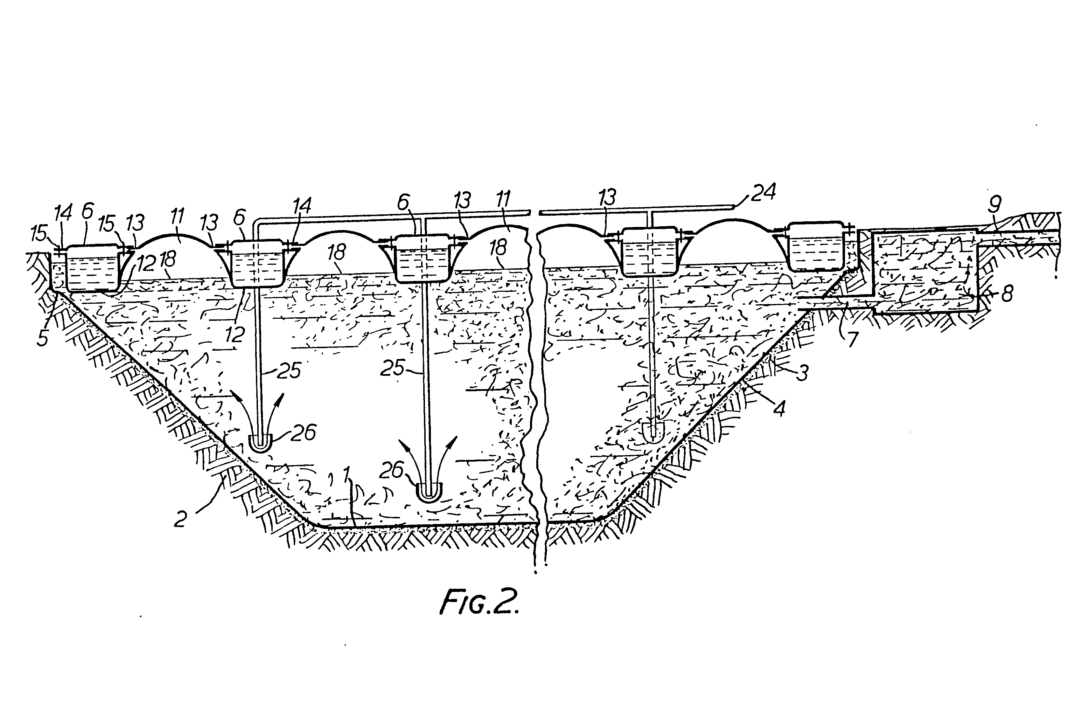

Referring to Figures 1 and 2, an anaerobic slurry digester shown therein comprises a trough 1 excavated in a body of earth 2 and rendered waterproof against seepage of water by provision of a sand lining 3 on which is placed a butyl rubber liner 4. The trough 1 is formed with opposed longitudinally extending shoulders 5 on which rest outermost elongate containers 6 filled with water when in use. An inlet duct 7 is provided for supply of sewage from a heat exchanger 8 having its own inlet pipe 9 for supply thereto of slurry from a stockyard.

The elongate containers 6 form part of a capping arrangement comprising a plurality of elongate capping members 10 juxtaposed in parallel relationship and comprising semi-cylindrical chambers 11 having flanges 12 at the free margins thereof and connected together by connecting means (not shown in the drawing) for ensuring that a stable structure is produced. In their upper regions, the chambers 11 are provided with outlets 13 which are connected to outlet 14 in the upper regions of the containers 6 and which are provided with valves 15 whereby the chambers 11 which can otherwise be placed in communication with each other through the containers 6 can by separated off from one another. The elongate containers 6 are provided with supply pipes 16 fitted with closure valves 17 for introduction thereinto and removal therefrom of water for providing water up to a variable level 18 therein (Figure 2).

Referring specifically to Figure 1, the heat exchanger 8 is of the passive type comprising, in addition to the inlet ducts 7 and 9, an outfall 19 for example to a lagoon or compost tank. The heat exchanger is formed with ducts 20 for through passage of fresh slurry and treated slurry which allow heat exchange to take place therebetween without direct contact occurring. A chamber 21 of the heat exchanger is formed with an outlet pipe 22 to a heat exchanger 23 for heating slurry from the digester and returning this slurry to the digester through a pipe 24. Rather than discharge the heated slurry at a single position into the digester, to achieve good stirring and agitation of slurry and to promote the digestion thereof, the pipe 24 supplies a plurality of descend pipes 25 located at intervals along the digester and terminating in cups 26 positioned adjacent the base of the digester. As a matter of convenience in constructing the overall digester, the pipes 25 pass through the capping arrangement at positions intermediate sections 27 of each elongate container 6.

Operation of the slurry digester takes place in the following manner. Slurry from a stockyard is fed through inlet duct 9, heat exchanger 8 and inlet duct 7 so as to substantially fill the container. Initially, the capping arrangement simply rests on the trough 1 by seating of the flanges of chambers 11 on the shoulders 5. At such time, the containers 6 will usually be empty. As the trough 1 fills up with slurry, a point will be reached at which the capping arrangement will be floated off the shoulders. Gas pressure build up in the chambers 11 will cause the capping arrangement to rise and from time to time, adjustment of the water level in the elongate containers 6 will have to be made to ensure that the level of the capping arrangement is satisfactory. It is expected that, in practice, it would be most satisfactory if periodic adjustment of the water level in the containers 6 is effected to maintain the gas pressure of 15.25cm water (146.5kg/m$^2$) under the capping arrangement. After a certain period of operation, the gas pressure build up may be sufficient to allow release of gas, mainly methane, from the chambers 11. This can either be burnt off or used as a source of fuel at a nearby site.

Scum removal from the surface of the slurry in the trough 1 can be achieved over bands extending across the trough by closing off valves 15 and removing chambers 11 from their positions in the capping arrangement or utilising access ports (not shown) thereby allowing ready access to the upper surface of the slurry for skimming purposes. However, scum formation is in fact minimised by the supply arrangement for heated slurry to the trough. As the heated slurry is supplied to the trough at positions disposed over the cross-section of the trough, turbulence is set up and this is responsible for minimising scum formation. The scum formation may be further reduced if the pipes 25 are formed with venturis (not shown) communicated with the upper region of the elongate containers 6 allowing methane to be drawn therefrom as a supply of gas which then bubbles upwardly through the slurry after it emerges from the pipes 22 at the cups 24. This venturi effect is equivalent to the use of a compressor for supplying gas under pressure to the interior of the trough 1.

The capping arrangement will generally be formed of plastics material, for example glass fibre reinforced plastics sprayed with polyerethane foam on the interior surfaces thereof as insulation to keep heat in. The capping arrangement may comprise capping members coloured white on the upper surface thereof to reflect heat incident thereon.

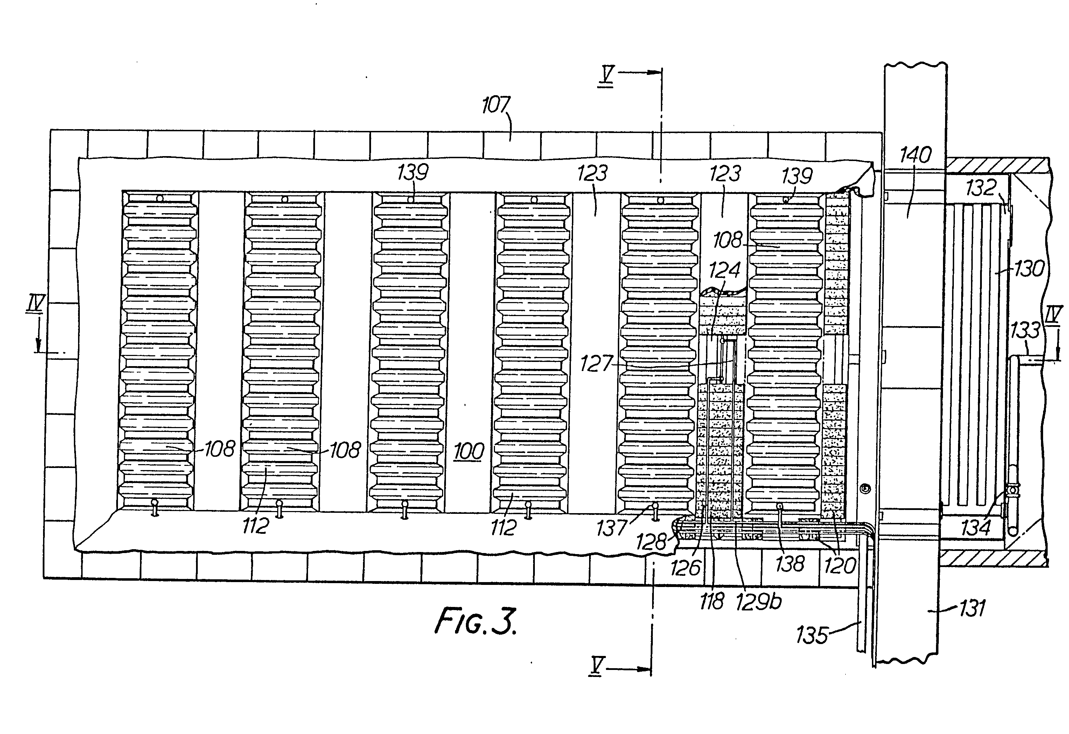

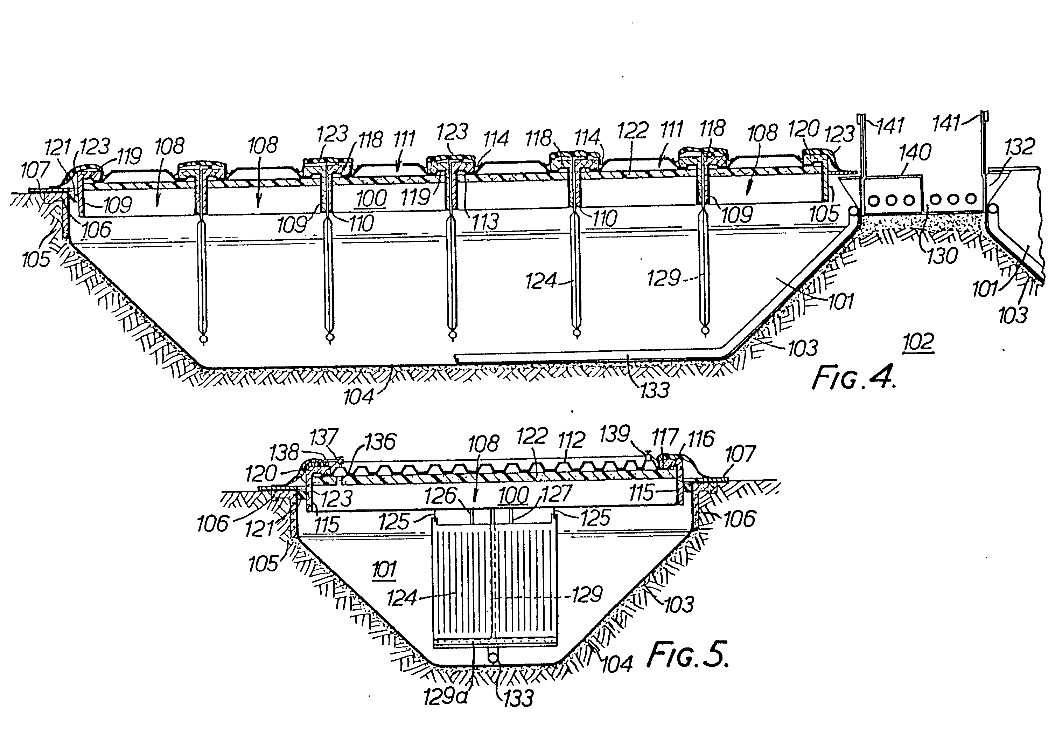

Referring next to Figures 3 to 5 of the accompanying drawings, a slurry digester arrangement comprises a pair of troughs 101 which, as will be described more fully hereinafter have a common service arrangement disposed therebetween. The use of a pair of digesters each comprising one of the troughs 101 allows one to be taken out of use and serviced, for example when the supply of slurry

0036915

-9-

for digestion drops, as in summer when cattle sheds will be less in use. The righthand trough 101 is in all respects identical with the lefthand trough 101, although it is not shown in full or in complete detail in Figures 3 and 4. Each trough 101 is excavated in a body of earth 102 and rendered proof against seepage of water by provision of a sand lining 103 on which is placed a butyl rubber liner 104. The troughs 101 are both of trapezoidal vertical cross-section over the major part of their depth terminating in vertical wall sections 105 formed by elongate concrete members 106 of inverted L-section set in the earth and providing a stable margin for the trough. A walkway 107 formed of concrete paving slabs extends around the mouth of each trough, the concrete paving slabs extending from the mouth of each trough over the elongate concrete members 106 and onto the surface of the earth 102.

Each trough 101 is covered by a capping arrangement 100 formed of a plurality of elongate sections 108 juxtaposed in parallel relationship. Each elongate section 108 is formed as a moulded fibre-reinforced plastics member pigmented white and smoothly finished externally so as to reflect heat incident thereon. A preferred material for the elongate sections 108 is glass fibre-reinforced acrylic-modified polyester resin, for example a resin of 60% by weight ester, 20% by weight methylmethacrylate and 20% by weight styrene compsition. Both surfaces of the elongate section are preferably coated with a vinyl silane finish to impart water repellent properties thereto. As can be seen better from Figure 4, the elongate sections 108 are each formed with lateral side walls 109 terminating in outwardly directed flanges 110 and connected together through upper wall sections 111 which comprise central regions 112 which can be seen from Figures 3 and 5 to be of substantially corrugated form and which are flanked by horizontal portions 113 which are formed with channels 114 adjacent the central regions 112. At their ends, the elongate sections 108 are formed in like manner to the lateral portions thereof, comprising vertical end walls 115 and horizontal portions 116 which incorporate channels 117 adjacent the terminal corrugations of the central regions 112. The individual elongate sections 108 are not connected directly to each other but instead are connected to each other through ballast members 118 of T-shaped cross-section disposed between the lateral side walls of the elongate sections and whose cross pieces 119 are modified to engage the channels 114 of the elongate sections to connect them together by an interlocking action. The ballast members 118 may conveniently be formed of stone aggregate concrete having a density of 140 lb per cubic foot. Because of their density, for ready handling, a large number of ballast members 118 of relatively narrow width are provided between each pair of elongate sections (Figure 3). The channels 114 in the elongate sections 108 which lie at the ends of the troughs 101 are occupied by suitably shaped portions of L-shaped ballast members 120 which in fact have a form equivalent to half of a T-shaped ballast member 18, which form is also possessed by the concrete members 106. Further L-shaped ballast members 120 are povided at the ends of the elongate sections, entering the channels 117 therein. The L-shaped ballast members 120 are spaced apart from the margins of the troughs and the entire capping arrangement comprising elongate sections 108 and ballast members 118 and 120 is intended to float on slurry in the trough and can be lifted therefrom by the pressure of methane gas generated during digestion of the slurry.

A sealing member 121 which does not inhibit the rising and falling of the capping arrangement is disposed between the L-shaped ballast members 120 and the vertical wall sections 105 of the trough.

It is desired to control the temperature of the slurry in the digester to be at the optimum value for digestion. Large temperature differences will generally result in microbial death. It is for this reason that the elongate sections are of white colouration so as to reflect incident sunlight. Moreover, thermal insulation is associated with the capping arrangement. Such thermal insulation takes the form of sections 122 of low density insulating material (e.g. foam strips of non water-absorbing type) extending lengthwise of elongate sections 108. The insulating material may be the product available under the name Kaytherm which is a foamed lava product enclosed in a plastics, for example polyethylene, cover The upper surfaces of the ballast members 118 and 120 are covered with flexible strips 123 of thermally insulating material which is here prefereably a white insulation filled weather resistant polyvinyl chloride casing.

Further control of temperature within the troughs is provided by a heat exchanger arrangement comprising a plurality of removable pressed steel heat exchangers 124 suspended from the capping arrangement by suspension devices 125. The heat exchangers 124 comprises inlet and outlet pipes 126 and 127 which are led over the tops of the ballast members 118 to main pipes 128 and 129 for supply and removal of warm water from the heat exchangers. Passing down the middle of each heat exchanger is a gas supply pipe 129 which bifurcates at the bottom of the heat exchanger into two branches each formed with a plurality of openings 129a for escape of gas therefrom. The gas supply pipes 129 are connected through a pipe 129b to a compressor to which gas is to be supplied by ducts from the upper regions of the capping arrangement in use (not shown).

In the region between the two troughs 101, is provided a passive heat exchanger 130 wherein heat exchange is to take place between incoming and outgoing slurry. Slurry is supplied to the heat exchanger 130 through a slurry channel 131 from cow cubicles and enters the troughs 101 through a slurry entry gate 132. Slurry is removed from the troughs through a pipe 133 having a shut-off valve 134 to the heat exchanger 130 which it leaves through a pipe 135. Pipe 133 allows for the removal of slurry from the tank either by pumping out or by slurry displacement.

The insulation sections 122 have gaps 136 formed therein to allow methane which collects in the elongate sections 108 to pass into corrugations thereabove, which corrugations are provided with valves 137 for withdrawal of methane therefrom to pipes 138 for removing the methane from the digesters. At the opposite end of each elongate section to that provided with a valve 137 is provided a venting valve 139. Both valves 137 and valves 139 are tailed down (not shown) below the insulation.

A boarded walkway 140 having barriers 141 which provide handrails and pipe support is provided over the heat exchanger 130.

Operation of the slurry digester takes place in the following manner. Slurry from a stockyard is fed through slurry channel 131 and heat exchanger 130 and entry gate 132 into the trough 101 so as substantially to fill it. Initially, the capping arrangement will rest within the trough with the bottom edge region of the L-shaped ballast member 120 resting on the upper part of the sloping walls of the trough. As the trough fills up with slurry, a point will be reached at which the capping arragement will be floated off. Warm water at a temperature monitored so as to be suitable for maintaining the slurry temperature at the optimum temperature for digestion thereof is fed into the heat exchangers 124 through the respective inlet pipes 126 and as digestion takes place, gas pressure build-up in the interior of the elongate sections 108 will cause the capping arrangement to rise as methane produced is collected thereunder. By forming the ballast members of 2243 kg/cu. meter concrete, the capping arrangment will be progessively lifted clear of the slurry undergoing digestion in three stages.

In the first stage there will be a build-up of the gas pressure from 0 to 10cm.WG. The capping arrangement will only have to have lifted 6" or 150 mm to have generated this pressure (the minimum gas operating pressure). In the second stage 10 to 15cm .WG will be achieved and gas storage will occur while the capping arrangement lifts a further 500 mm. Gas may be removed from the capping arrangement through the valves 137 during this stage so as to maintain a gas pressure in the range 10 to 15cm .WG. In a third and final stage when gas pressure increases to 15 to 17.5cm.WG, and if this is to be achieved in the absence of planned gas removal, the capping arrangement continues to lift until it vents from its rim. In this way safety blow-out will be achieved. The modified T-shaped of the ballast members 118 is such as to allow this break up of the capping arrangement as a safety measure by application of sufficient gas pressure from below.

Scum removal from the surface of the slurry in the trough 101 can be achieved over bands extending across the trough by closing off valves 137, opening valves 139, removing ballast members 118 at the margins of the elongate section 108 which is to be removed and then removing the elongate section 108 concerned thereby allowing ready access to the upper surface of the slurry for skimming purposes. However this is a somewhat time consuming operation, especially as dismantling of the various pipes passing over the ballast members 118 is required. Scum formation is in fact minimised by supply of methane under pressure through the gas supply pipes 129 into the bottom of the digester since as the methane emerges from the openings 129a, it bubbles upwardly through the slurry.

The methane removed from the elongate sections 108 of the capping arrangement can be burnt off or used as a source of fuel at a nearby site. In particular it may be employed to heat water to be supplied to the heat exchangers 124. The methane may alternatively be burnt in an internal combustion engine connected to a generator thereby yielding electrical energy which may be used on a farm site, _inter alia_ for operating a compressor for compressing a portion of the methane for return to the troughs through the pipe.

A capping arrangement according to this invention is modular in its construction and thus has the advantage that capping arrangements of any desired size can readily be built up from the basic elements. Moreover, by being capable of shutting off one from another, individual sections can be closed off when it is desired to remove scum or when a section has become defective.

By being provided at and below ground level, a slurry digester according to this invention is particularly suitable for use in hydroponic cultivation of plants. The hydroponic beds can be disposed over the capping arrangement using the contours thereof to define individual beds. Some heat loss will unavoidably occur from the slurry digester through the cap and this will be of direct benefit to the beds undergoing cultivation and which may be supplied with slurry from the digester as a source of organic nutrients.

CLAIMS:

1.      A capping arrangement for an anaerobic slurry
digester of substantially rectangular horizontal cross-
section, the capping arrangement being intended to float
on slurry in the digester and be lifted from the slurry
by gas under pressure produced in the digester and comprising a plurality of ballasted elongate capping members
arranged side-by-side, characterised in that the capping
arrangement comprises an array of capping members (10,
108) of like width in a modular arrangement, which capping
members are of inverted channel form defining therebetween channels which are upwardly open, the capping members
(10,108) being formed with gas outlets (13,137) in upper
regions thereof and in that ballast means (6,118) are
positioned in said upwardly open channels and act to keep
said capping members (10,108) in their side-by-side
arrangement when positioned on said digester and floating
on a body of slurry and when being lifted therefrom by gas
under pressure.

2.      A capping arrangement according to Claim 1,
characterised in that said capping members (10) are of
substantially hemi-cylindrical cross-section and have at
their lateral margins flange sections (12) at which the
adjacent capping members adjoin, and the ballast means comprises
elongate tanks (6) resting on said flange sections (12)
and being provided with means for supply of water thereto
and removal of water therefrom.

3.      A capping arrangement as claimed in Claim 2, characterised in that said gas release outlets are connected
by duct means with upper regions of said ballast tanks (6).

4.      A capping arrangement as claimed in Claim 1,
characterised in that the capping members (108) have
upstanding lateral walls (109) formed with shoulders (113)
on which rest one side of the head (119) of solid ballast
members (118) substantially of letter T-shape in transverse cross-section.

5.      A capping arrangement according to Claim 4,
characterised in that said ballast members (118) are formed of concrete.

6.      A capping arrangement according to Claim 4 or 5, characterised in that said shoulders are formed with channels (114) running lengthwise thereof and the heads (119) of said ballast members are correspondingly shaped whereby the ballast members (118) serve to hold together adjacent capping members (108) by interlocking therewith.

7.      A capping arrangement according to any one of Claims 4 to 6, characterised in that an upper wall (111) of each capping members (108) extending lengthwise thereof between said shoulders is substantially of corrugated form.

8.      A capping arrangment according to any one of the preceding claims, characterised in that the capping members (10,108) are formed of glass fibre reinforced plastics material coloured white at least on upper surfaces thereof.

9.      An anaerobic slurry digester which comprises a basin of substantially rectangular horizontal cross-section and having a substantially horizontal upper periphery, inlet means for supply of slurry to the basin below said periphery, means for removal of sludge from the basin and a covering for the basin, characterised in that the covering comprises a capping arrangement according to any one of the preceding claims and sized to lie within the basin (1,101) with its outer margin adjacent the periphery thereof, disposed in an upper region of the basin.

10.     A slurry digester according to Claim 9, characterised in that said basin is an excavated trough in the earth.lined by a liner (4,104) so as to prevent water seepage therefrom.

11.     A slurry digester according to Claim 9 or 10, the capping arrangement being a capping arrangement according to Claim 2 or 3, characterised in that the basin comprises a shoulder (5) extending therearound below the upper periphery thereof on which can rest in the case of shoulders (5) at the ends of the basin, the endmost elongate tanks (6) and in the case of shoulders extending lengthwise of the basin, the ends of the elongate tanks (6).

12.     A slurry digester according to any one of Claims 9 to 11, characterised in that it additionally comprises indirect heat exchanger means (8,130) for achieving heat exchange between slurry being fed to the basin and sludge being removed therefrom, in use.

13.     A slurry digester according to any one of claims 9 to 12, characterised in that it additionally comprises heating means for heating of slurry in the basin, which heating means comprises duct means (22) branched off from said sludge removal means, a heating arrangement (23) associated with said duct means and further duct means (24) for return of heated sludge to the basin.

14.     A slurry digester according to Claim 13, characterised in that said duct means (24) for returning sludge to the slurry digester comprises a plurality of sludge feed pipes (25) comprising descender portions extending to a lower region of the basin (1,101) at discrete intervals.

15.     A slurry digester according to Claim 14, wherein the capping arrangement is a capping arrangement as claimed in Claim 3, characterised in that the descender pipes (25) pass through the elongate tanks (6), which descender pipes have venturis associated therewith for drawing gas from said upper regions of the elongate tanks (6) thereinto.

16.     A slurry digester according to Claim 14 or 15, characterised in that said descender pipes (25) open into cup-shaped members (26) for upward deflection of matter issuing therefrom in use.

17.     A slurry digester according to any one of Claims 9 to 12, characterised in that it additionally comprises heating means for heating of slurry in the basin, which heating means comprises indirect heat exchangers (124) suspended below the capping arrangement (108), which heat exchangers have supply ducts (126) for supply of warm water thereto and outflow ducts (127) for removal of water therefrom.

18.     A slurry digester according to any one Claims 9 to 15 and 17, characterised in that it comprises ducts for withdrawal of gas from upper regions of the capping members (108), compressor means for said gas and return ducts (129) for return of gas to a lower region of the basin (101), which return ducts are formed at said lower region with a plurality of small apertures (129a) for ejection of gas under pressure.

19.     A slurry digester according to Claims 17 and 18, characterised in that each heat exchanger (124) is in the form of a pressed steel panel down a central portion of which passes a said return duct (129) and along a bottom region of which branches of the return duct formed with said small apertures (129a) are provided.

FIG.I.

FIG.2.

0036915

FIG.3.

FIG. 4.

FIG. 5.

European Patent
Office

**EUROPEAN SEARCH REPORT**

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | GB - A - 1 411 659 (H. HALL) <br><br> * Page 1, lines 36-84; page 2, line 55 - page 3, line 52; page 4, line 104 - page 5, line 48 * <br><br> -- | 1,9-12, 15,18 | C 02 F  3/28 <br> B 65 D 88/76 <br>        88/34 <br> A 01 C  3/02 |
| A | GB - A - 471 003 (J. MILLS et al.) | | |
| A | DE - C - 640 367 (BAMAG) | | |
| A | GB - A - 518 353 (P.A. LEITCH et al.) <br><br> ---- | | **TECHNICAL FIELDS SEARCHED (Int. Cl.³)** <br><br> C 02 F  3/28 <br> B 65 D 88/76 <br>        88/34 <br> C 12 M  1/00 <br> A 01 C  3/02 |

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search <br> The Hague | Date of completion of the search <br> 25-11-1980 | Examiner <br> TEPLY |
|---|---|---|

EPO Form 1503.1  06.78